# EUROPEAN PATENT APPLICATION

(11) **EP 1 088 562 A1**
(43) Date of publication of application: **04.04.2001**
(21) Application number: 99810876.5
(22) Date of filing: 29.09.1999
(51) Int. Cl.: A61L 9/12

(54) **A device for the controllable transfer of a liquid and an apparatus for dispensing transferred liquids**

(71) Applicant: Givaudan SA, 1214 Vernier-Genève (CH)
(72) Inventor: Hart, Gerald, Surbiton, Surrey KT5 8BD (GB); Naish, Guy, Narborough, Leicester LE9 5DD (GB)
(74) Representative: Patentanwälte Schaad, Balass, Menzl & Partner AG

(57) **Abstract**

The device (1) which allows the controllable transfer of a liquid (21), such as a perfume, a pharmaceutical agent, a masking agent, an insecticide or an organoleptic substance, through an outlet opening (22) of a container (22) comprises a holder (10) with a fitting (11) being designed to embrace a capillary medium (5) and to fit into the opening (22) in such a way that liquid is only transferable through the opening (22) by the capillary medium (3). The holder (10), adjacent to the fitting (11), further comprises a sleeve (13) which further embraces the capillary medium (3) at least at a length which is chosen in such a way that, in a first vertical position of the sleeve (13) liquid (21) has access and in a second, inverse, vertical position of the sleeve (13) liquid (21) has no access to the capillary medium (3). The apparatus comprises at least one device (1) installed on a rotor (9) .

## Description

The present invention relates to a device for the controllable transfer of a liquid and an apparatus for dispensing transferred liquids with at least one of said devices according to claim 1 respective claim 7.

### BACKGROUND OF THE INVENTION

In order to transfer liquids it is known to use capillary action which is dependent on the cohesion forces within the liquid and the adhesion forces of the liquid to a porous capillary medium comprising small channels for the transfer of the liquid. Such a capillary medium, being introduced through an opening into a container, can therefore transfer a liquid out of said container by means of capillary action. The transferred liquid may be used to distribute chemical substances to the ambient air in order to mask or generate an odour, to evoke a medical or organoleptic effect or to affect insects.

In order to stop the transfer of liquid the capillary medium must be removed or covered which is inconvenient and may cause further distribution of remainders of the liquid on the cover or the removed capillary medium. The use of two or more liquids increases the inconvenience accordingly.

The present invention is therefore based on the object of specifying a device, which allows an easily controllable transfer of a liquid through a capillary medium and an apparatus for dispensing transferred liquids with at least one of said devices.

### SUMMARY OF THE INVENTION

The above and other objects of the present invention are achieved by a device and an apparatus as specified in claim 1 respective 7.

The inventive device allows the controllable transfer of a liquid, such as a perfume, a pharmaceutical agent, a masking agent, an insecticide or an organoleptic substance, by a first capillary medium such as a scrib-rod from a container preferably to a second capillary medium such as a gauze. The first capillary medium being inserted into a holder comprising a fitting being designed to embrace the capillary medium and to fit into an opening of the container. Said holder further comprising a preferably non-porous sleeve which further embraces the capillary medium at least at a length which is chosen in such a way, that, in a first vertical position of the sleeve, liquid has access and in a second, inverse, vertical position of the sleeve the liquid has no access to the capillary medium.

The inventive device is therefore easily controllable by turning it from a first to a second position.

In a preferred embodiment of the device the end of the sleeve extends to a side-wall of the container in such a way that in a third, horizontal position of the container access of the liquid to the capillary medium is prevented as well. This embodiment is used for an apparatus comprising two inventive devices which in positions one and two are activated alternatively and which are both deactivated in the third position. A further advantage of this embodiment is, that two or more liquids can be diffused separately.

In a further embodiment the first capillary medium is connected to a second capillary medium such as a gauze or an extended portion of the first capillary medium, from which the transferred liquid is diffused preferably under the support of an the air flow generated by fan.

The inventive apparatus, which allows dispensing of transferred liquids, comprises at least one inventive device as described above being rotatably mounted on an axis of a stator between at least a first vertical and a second thereto inverse position.

In a principal embodiment the apparatus comprises two inventive devices arranged towards each other along the same axis on a rotor with the second capillary medium oriented towards the centre of the rotor in such a way that in a first position of the rotor only one of the devices and in the second position the other one of the devices transfers the liquid.

The apparatus is preferably equipped with a control unit comprising a sensor for detecting the position of the rotor. Said control unit being capable to actuate the air flow generator and/or to control the air flow depending on the position of the rotor.

In the third position the diffusion of the liquid is therefore immediately stopped since liquid is no longer transferred by the first capillary medium and no longer diffused from the second capillary medium.

In a further embodiment the apparatus can be fully automated.

### BRIEF DESCRIPTION OF THE DRAWINGS

Some of the objects and advantages of the present invention have been stated, others will appear when the following description is considered together with the accompanying drawings, in which:
- Fig. 1: shows a holder 10 and a first capillary medium 3, separated from each other;
- Fig. 2: shows the holder 10 with the installed capillary medium 3;
- Fig. 3: shows a container 20 with an opening 22 suitable for inserting the holder 10;
- Fig. 4: shows the container 20 with the holder 10 and the capillary medium 3 installed;
- Fig. 5: shows the container 20 of Fig. 4 turned upside down;
- Fig. 6: shows a modified container 201 comprising two reservoirs 204a, 204b;
- Fig. 7: shows the first capillary medium 3 connected to a second capillary medium 5;
- Fig. 8: shows the arrangement of Fig. 7 installed in a container 20 of a first and a second inventive device 1x respective 1y;
- Fig. 9: shows the two inventive devices 1x, 1y of Fig. 8 in horizontal alignment and a third, vertically aligned inventive device 1z transferring liquid through the capillary media 3 and 5 and
- Fig. 10: shows an inventive apparatus with the devices 1x, 1y of Fig. 8 installed.

### DETAILED DESCRIPTION OF THE PREFERRED EMBODIMENTS

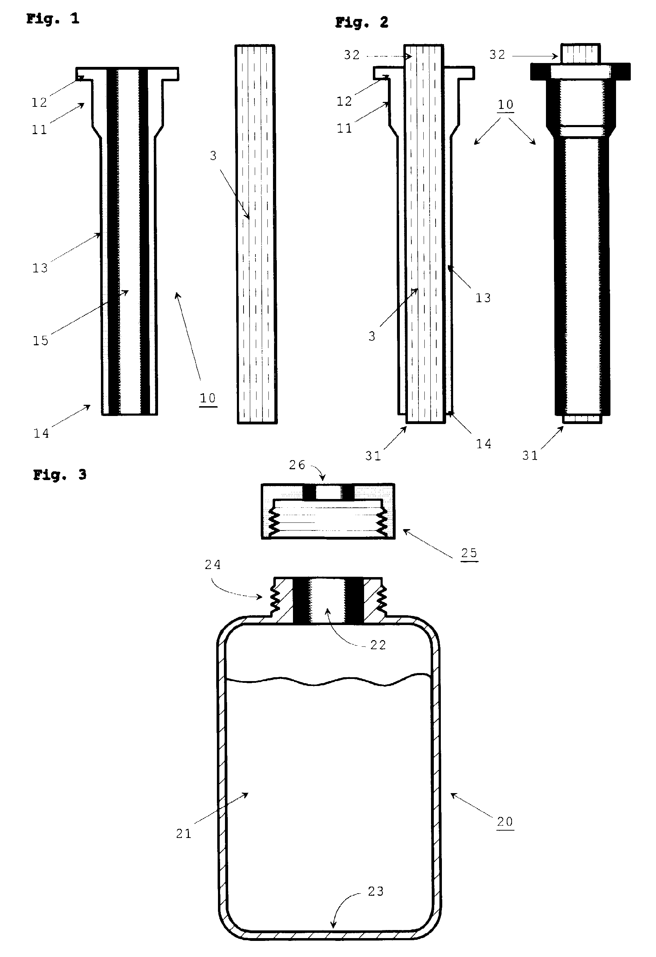

Fig. 1 shows a first capillary medium 3 and a holder 10 (in a sectional view) comprising a fitting 11 with a flange 12; said fitting 11 being connected to a sleeve 13. Fitting 11 and sleeve 13 build a tube with a channel 15 into which the capillary medium 3 can be inserted.

Fig. 2 shows the holder 10 with the installed capillary medium 3. The length of the capillary medium 3 is chosen in such a way that it is longer than fitting 11 and sleeve 13 combined. When installed, one end 31 of the capillary medium 3 will extend out of the sleeve 13 respective the end 14 of the sleeve 13 and the other end 32 will extend out of the fitting 11, which tightly seals the opening 22 after the installation.

Fig. 3 shows in a sectional view a container 20 with an opening 22 and a corresponding fitting 24 which is suitable to receive the fitting 11 of the holder 10 in such a way that the sleeve 13 is inserted into the container 20 and the flange 12 rests on the fitting 24 of the container 20 which is filled with a liquid 21 such as a perfume, a pharmaceutical agent, a masking agent, an insecticide or an organoleptic substance. Further shown is a cover 25 with an opening 26 through which the capillary medium 3 can be guided. The cover 25 can be screwed onto the fitting 24 of the container 20.

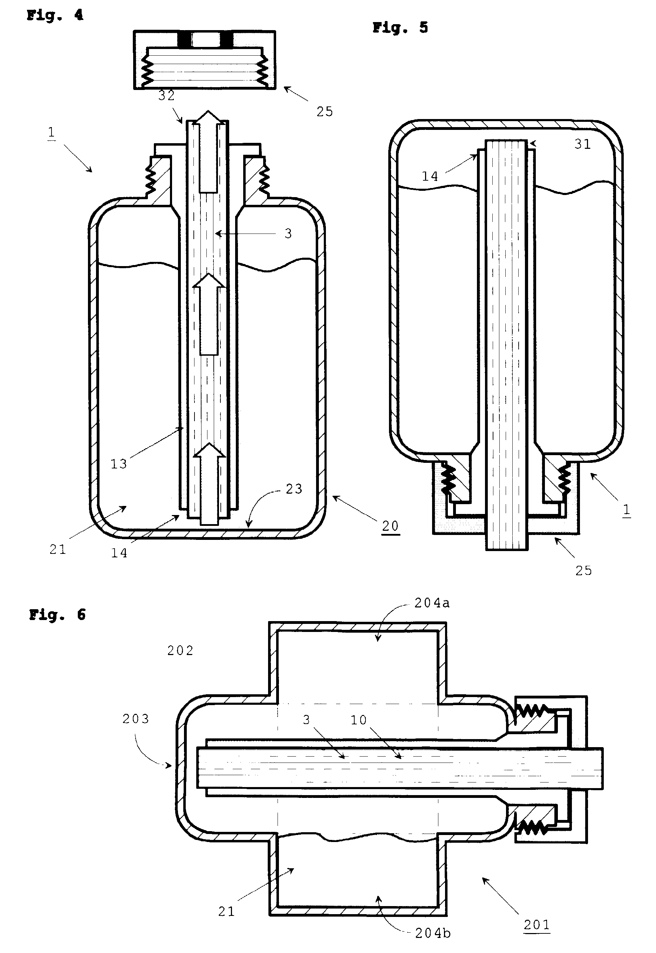

Fig. 4 shows the container 20 with the holder 10 and the capillary medium 3 installed. Due to the tight sitting of the fitting 11 in the opening 22 and the tight sitting of the capillary medium 3 in the channel 15 of the holder 10 the liquid 21 is only transferable through the opening 22 through the capillary medium 3 by capillary action. The liquid 21 can enter only at the end 31 of the capillary medium 3 respective at the end 14 of the sleeve 13 since the sleeve 13 shields the remaining part of the capillary medium 3, introduced into the container 20, against the liquid 21. The end 14 of the sleeve 13 of the installed holder 10 preferably extends to a region close to the bottom 23 of the container 20.

Therefore, when turning the container 20 upside down, as shown in Fig. 5, the liquid 21 has no access to the capillary medium 3 which is shielded by the sleeve 13. Only in case that the container 20 were filled more than 95%, liquid could reach the capillary medium 3. However the capillary medium 3 and the sleeve 13 could also be extended to the bottom 23 of the container 20, comprising in there narrow channels for the liquid 21 to pass through to the capillary medium 3.

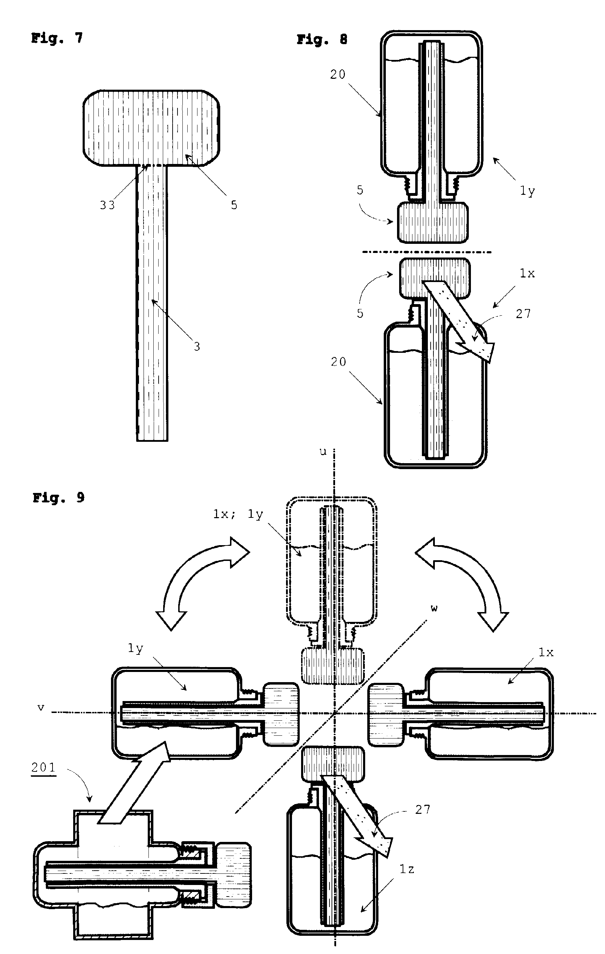

Fig. 6 shows a modified container 201 comprising two reservoirs 204a respective 204b at the side-wall 202, which receive the liquid 21 whenever the container 201 is turned into horizontal alignment. A clockwise rotation of the container 201 will cause the liquid 21 to enter reservoir 204b. A counter-clockwise rotation will cause the liquid 21 to enter reservoir 204a. The liquid 21 will therefore not be in contact with the capillary medium 3 in both horizontal orientations of the container 21. The reservoirs 204a, 204b may also be part of a cylindrical extension of the side-wall 202. The modified containers 201 are recommended for the use in arrangements comprising three or more inventive devices 1x, 1y, 1z. In the arrangement shown in Fig. 9 modified containers 201 could therefore advantageously be installed.

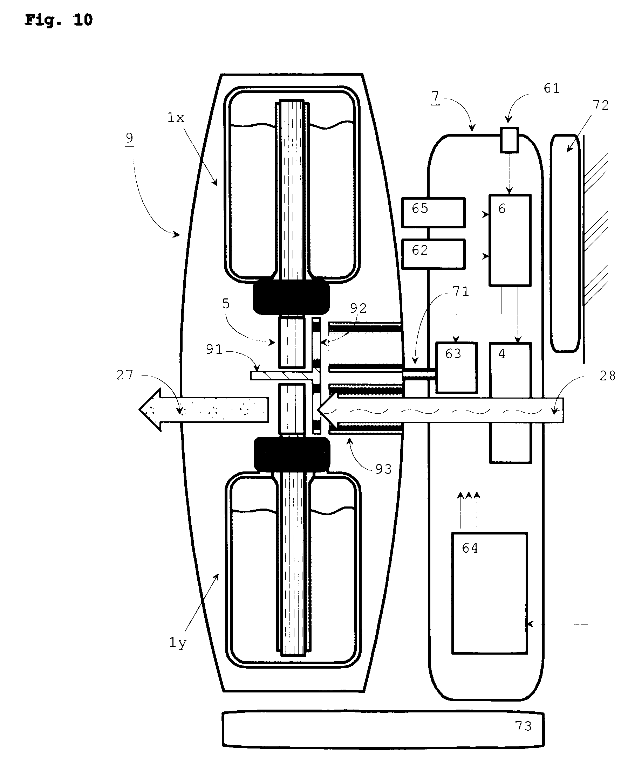

Fig. 7 shows the first capillary medium 3 connected to a second capillary medium 5 such as a gauze or an extended portion of the first capillary medium 3 which preferably consists of a porous synthetic material from which the transferred liquid 21 is exposed to the air or to the air flow 28 generated by an air flow generator 4 (see Fig. 10). From the first or second capillary medium 3; 5 the diffused liquid 27 (see Fig. 8, 9 or 10) is forwarded to the environment.

Fig. 8 shows the arrangement of Fig. 7 installed in a container 20 of a first and a second inventive device 1x respective 1y. The first device 1x is oriented as the device 1 shown in Fig. 4. As explained above in this orientation liquid 21 is transferred by the capillary medium 3. The second device 1y is oriented as the device 1 shown in Fig. 5 preventing the transfer of liquid 21.

Fig. 9 shows the two inventive devices 1x, 1y of Fig. 8 in horizontal alignment along an axis v and a third device 1z in vertical alignment along an axis u. The third device 1z is transferring liquid 21 through the capillary media 3 and 5 while no liquid is transferred by the horizontally aligned devices 1x, 1y, which contain only a small amount of liquid 21. Turning the arrangement around axis v by 90°, will cause transfer of liquid 21 to stop in device 1z as well. However, turning the arrangement around an axis w, which is perpendicular to the axis u and v, to the left or to the right by 90° will start transfer of liquid 21 in one of the devices 1x respective 1y while transfer of liquid 21 in the device 1z will cease.

Adding two inventive devices 1 aligned along axis w will extend the arrangement to five devices 1 which can be turned around axis v or w into corresponding positions where liquid 21 is transferred in the corresponding devices 1.

In order to allow the use of larger volumes of liquid 21 the devices 1x, 1y and 1z can be replaced by devices comprising a modified container 201 as shown in Fig. 6.

Of course the vertical and horizontal alignment need not be exact and can vary in a broad angle depending on the percentage of liquid 21 in the containers 20; 201 and the form (bent, straight) and length of the sleeve 13.

It is also possible to use a single inventive device 1 preferably in a housing which can be put on a surface in at least two positions in such away that in both positions (inverse and not inverse) the device 1 is at least approximately vertically aligned.

Fig. 10 shows an inventive apparatus for dispensing liquids 21 comprising a stator 7 which, on a axis 71, carries a rotor 9 with two axially aligned inventive devices 1x, 1y. Possible is also the installation of only one device 1x in the apparatus. In the present position only the lower device 1y is transferring liquid 21 through the first and second capillary medium 3; 5 from where it is forwarded to the environment by a flow of air 28 being generated by a fan 4, which is preferably located in the stator 7. The air flow 28 is preferably guided in channels 93 in the rotor 9 and in the stator 7 in order to maximise the efficiency. The second capillary media 5 of the first and the second device 1x, 1y may be framed and isolated from each other by a frame 91 comprising openings 92 for the air flow 28. The rotor 9 can now be turned between at least a first position, e.g. as shown in Fig. 10 and a position in which the rotor 9 is turned by 180°. In the first position, liquid 21 from the first device 1y is transferred and diffused, while no liquid 21 is transferred in the second device 1x. In the second position liquid 21 is transferred in the second device 1x while no liquid 21 is transferred in the first device 1y. The liquid 21 to be diffused can therefore easily be selected by turning the rotor 9. In case that no liquid 21 should be diffused the devices 1x and 1y are brought into a third position where they are horizontally aligned. In case that this third position is used in the apparatus, then preferably the containers 201 shown in Fig. 6 are installed.

The positions of the rotor 9 are preferably detected by a sensor 62 which is connected to a control unit 6 which is controlling the fan 4 and/or a motor 63 which in a preferred embodiment is used to turn the rotor between positions. In case that the rotor 9 reaches the third position driven by the motor 63 or manually the fan 4 is switched off by the control unit 6 since no liquid is transferred in this position. In a further embodiment, depending on the input from a sensor 65, which measures temperature and/or humidity, the control unit 6 adjusts the air flow generated by the fan 4 or actuates fan 4 and rotor 9 in a suitable way.

The electrical devices in the apparatus are connected to a power supply 64. In order to control the apparatus preferably one or more control buttons 61 (mains switch etc.) are installed. The apparatus may also be programmable and equipped with a timer.

The apparatus may rest on a support 73 or may be installed on the wall by corresponding means 72.

## Claims

1. A device (1) for the controllable transfer of a liquid (21) stored in a container (20; 201) through an outlet opening (22) of said container (20; 201) with a holder (10) for a capillary medium (3) which extends into a region close to the bottom (23) of the container (20; 201), said holder (10) comprising a fitting (11) being designed to embrace the capillary medium (3) and to fit into the opening (22) in such a way that liquid is only transferable through the opening (22) by the capillary medium (3), **characterised in that** the holder (10), adjacent to the fitting (11), further comprises a sleeve (13) which tightly embraces the capillary medium (3) up to or close to its end (31) so that the device (1) can be turned between at least a first position in which liquid (21) is transferred while contacting the end (31) of the capillary medium (3) and at least a second position in which the end (14) of the sleeve (13) extends out of the liquid (21) thereby preventing contact of the liquid (21) to the capillary medium (3).

2. Device (1) according to claim 1, wherein the capillary medium (3), which is extending out of the fitting (11), is connected to a second capillary medium (5) such as a gauze or an extended portion of the first capillary medium (3), from which the transferred liquid (21) is exposed to the air or to the air flow generated by an air flow generator (4) and evaporated.

3. Device (1) according to claim 1 or 2, wherein the container (201) is equipped with at least one reservoir (204a, 204b) which is suitable to receive the liquid (21) when the container (201) is turned into a third, approximately horizontal position.

4. Device (1) according to claim 1, 2 or 3, wherein the opening (22) can be closed by a cover (25) comprising an opening (26) suitable for inserting the capillary medium (3).

5. Device (1) according to one of the claims 1-4, wherein the fitting (11) comprises a flange (12) which extends over the edges of the opening (22).

6. Device (1) according to one of the claims 1-5, wherein the liquid (21) is a perfume, a pharmaceutical agent, a masking agent, an insecticide or an organoleptic substance.

7. Device (1) according to one of the claims 1-6, wherein the capillary medium (3) is a scrib-rod.

8. Apparatus for dispensing at least one liquid (21), comprising at least one device (1x, 1y, 1z) according to claim 2 for the controllable transfer of a liquid (21) through an outlet opening (22) of a container (20; 201) to a second capillary medium (5) such as a gauze, said device (1x, 1y, 1z) being rotatably mounted between at least a first position in which liquid (21) is transferred and a second position in which transfer of liquid (21) is prevented.

9. Apparatus according to claim 8 with a rotor (9) which can be turned around at least a first axis (w) in such a way that for two devices (1x, 1y), being installed on a second axis (v) perpendicular to the first axis (w), in a first position of the rotor (9) liquid (21) is transferred only in the first device (1x) and that in a second position of the rotor (9) liquid (21) is only transferred in the second device (1y).

10. Apparatus according to claim 9 with a third device (1z) being installed on a third axis (u) being perpendicular to the first and the second axis (v, w) in such a way that in a in a first position of the rotor (9) liquid (21) is transferred only in the first device (1x), in a second position of the rotor (9) liquid (21) is only transferred in the second device (1y) and in a third position of the rotor (9) liquid (21) is only transferred in the third device (1z).

11. Apparatus according to claim 9 or 10 with the rotor (9) being rotatable around the first axis (w) and the second axis (v) with at least a fourth and/or a fifth device (1) being installed on the first axis (w) in such a way that in a fourth position of the rotor (9) liquid (21) is transferred only in the fourth device (1) and that in a fifth position of the rotor (9) liquid (21) is only transferred in the fifth device (1) .

12. Apparatus according to claim 9, 10 or 11 with the second capillary medium (5) of the installed devices (1, 1x, 1y, 1z) being oriented towards the crossing point of the first and the second axis (v, w).

13. Apparatus according to one of the claims 9-12, comprising an air flow generator (4) such as a fan which is generating an air flow towards at least the second capillary medium (5) of the device (1, 1x, 1y, 1z) whose first capillary medium (3) is exposed to the liquid (21).

14. Apparatus according to one of the claims 9-13, with a control unit (6) comprising a switch (61) for actuating the air flow generator (4) and/or comprising a sensor (62) for detecting the position of the rotor (9) said control unit (6) being capable to actuate the air flow generator (4) and/or to control the air flow depending on the position of the rotor (9).

15. Apparatus according to claim 14, said control unit (6) being programmable and capable to control a motor (8) which is used to turn the rotor (9) between the operating positions.

16. Apparatus according to one of the claims 9-15 comprising a stator (7) comprising the air flow generator (4) and a bearing for the axis (71) of the rotor (9) which carries at least one, preferably up to five devices (1x, 1y, ...) according to claim 6.

17. Apparatus according to one of the claims 9-16 wherein the liquid (21) is a perfume, a pharmaceutical agent, a masking agent, an insecticide or an organoleptic substance.
